# EUROPEAN PATENT SPECIFICATION

(11) **EP 1 215 279 B1**
(45) Date of publication and mention of the grant of the patent: **31.12.2008**
(21) Application number: 01127456.0
(22) Date of filing: 27.11.2001
(51) Int. Cl.: C12M 1/00, C12G 1/00, A23N 1/00

(54) **Device for processing organic material, in particular fruit or vegetables, in a liquid phase**
Verfahren zur Behandlung von organischen Materialien, insbesondere von Früchten und Gemüsen, in einer flüssigen Phase
Appareil pour le traitement de matière organique, plus particulièrement des fruits ou végétaux, dans une phase liquide

(30) Priority: 13.12.2000 IT RM000232 U
(43) Date of publication of application: 19.06.2002
(73) Proprietor: Valentini, Valentino, I-00128 Roma (IT); D'Ettole, Antonio, 70031 Andria (IT)
(72) Inventor: Valentini, Valentino, 00128 Roma (IT)
(74) Representative: Franzolin, Luigi

(56) References cited:
- CH-A- 365 364
- DE-A- 4 035 228
- FR-A- 1 086 159
- US-A- 2 864 746
- US-A- 4 556 587
- US-A- 4 737 278

## Description

The present invention relates to a device for processing organic material, in particular fruit or vegetables, in a liquid phase.

More specifically, the present invention relates to a device for effecting a chemical process whereby fruit and/or vegetables are maintained in close contact with a liquid phase, and to which the present invention refers purely by way of example.

Fruit and vegetables are normally subjected to chemical processing, such as fermentation and retting, which requires relatively prolonged, close contact between organic material, such as fruit and vegetables, and a liquid phase. In the case of fermentation, the liquid phase is defined by the must produced at the preliminary pressing stage, and, in the case of retting, by a liquid such as ethanol or hexane, which are particularly suitable for extraction.

Fermentation and retting are normally conducted in a device comprising a tank defining a chamber containing both the organic material and the liquid phase, which are kept in contact with each other inside the tank until the ongoing chemical process reaches a desired stage. In other words, during the chemical process, the fruit and vegetables release organic components to the liquid phase; and, to interrupt the chemical process, the liquid phase, which constitutes the end or semifinished product, is extracted from the tank and separated, e.g. by tapping or filtration, from the solid phase, i.e. the organic material remains, which are then also extracted from the tank.

Devices of the above type have the drawback of failing to keep the organic material fully immersed in the liquid phase, owing to the lower specific weight of the organic material, which tends to float in the liquid phase.

As a result, the organic material is partly exposed to air and inevitably undergoes oxidation and/or aerobic fermentation, which deteriorates it and impairs its organoleptic properties.

Moreover, the buoyancy of the organic material with respect to the liquid phase results in incomplete chemical processing, through failure to keep the organic material in constant, close contact with the liquid phase resulting in some of the organic material components not being released to the liquid phase, and so being dispersed in the air, as opposed to forming a solution with the liquid phase. This is particularly undesirable in the case of components responsible for the odour of the end product, and which, being particularly volatile, are readily dispersed in air if not immersed in the liquid phase.

To eliminate the drawback posed by the buoyancy of the organic material, substantially two types of device have been devised. A first comprises a tank; and a movable member housed inside the tank to stir the organic material and prevent it from remaining in contact with air for relatively prolonged periods of time.

A second comprises a tank; a liquid phase recirculating conduit; and a pump connected to the recirculating conduit to spray the floating organic material continuously and so prevent contact between the organic material and air.

Both the above types of device, however, on the one hand, only provide for limiting the aforementioned drawbacks, and, on the other, subject the organic material to undesired mechanical stress, which has a negative effect on the quality of the end product by promoting the extraction of components from the organic material, e.g. from the seeds of fruit and vegetables.

A device has also been proposed, in which the air inside the tank would be replaced by an inert gas, such as nitrogen. While solving the problems posed by oxidation and aerobic fermentation of the organic material, such a device would still fail to prevent the loss of certain substances which are vital to the quality of the end product.

It is an object of the present invention to provide a device for processing fruit, in a liquid phase, and the technical characteristics of which provide a straightforward, low-cost solution to the drawbacks typically associated with the known state of the art.

According to the present invention, there is provided a device for processing fruit in a liquid phase; the device comprising a tank having a wall and a hatch defining a chamber for containing said organic material and said liquid phase; and the device being characterized by comprising a partition member, which is housed inside said chamber, divides the chamber into a first compartment housing the organic material and the liquid phase, and a second compartment only housing the liquid phase, and comprises a number of openings permitting passage of the liquid phase between the first and the second compartment.

In one embodiment of the present invention, the partition member comprises a cage having a number of holes; said first compartment being defined inside said cage, and said second compartment being defined between the cage and the wall and hatch of said tank.

Two preferred, non-limiting embodiments of the present invention will be described by way of example with reference to the accompanying drawings, in which:
Figure 1 shows a longitudinal section, with parts removed for clarity, of a first preferred embodiment of the device according to the present invention;
Figure 2 shows a section along line II-II of the Figure 1 device;
Figure 3 shows a longitudinal section, with parts removed for clarity, of a second preferred embodiment of the device according to the present invention;
Figure 4 shows a section along line IV-IV of the Figure 3 device.

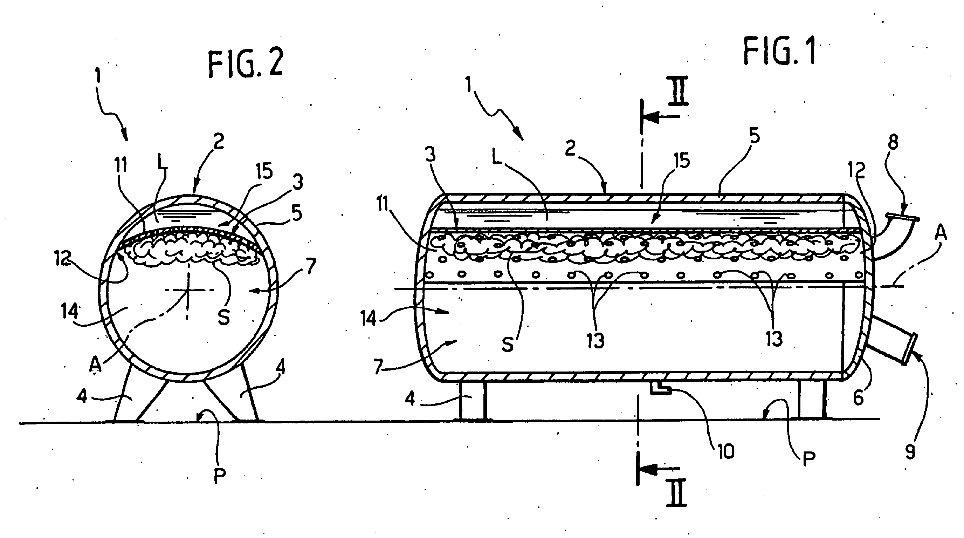

Number 1 in Figures 1 and 2 indicates as a whole a device for processing organic material S, such as fruit or vegetables, in a liquid phase L. In the following description, the term "processing" is intended to mean a fermentation or retting process; "organic material S" refers indifferently to fruit and/or vegetables; and "liquid phase L" is intended to mean both the must produced by pressing/squeezing the fruit and/or vegetables in the fermentation process, and the ethanol, hexane, etc. in the retting process.

Device 1 comprises a substantially cylindrical tank 2 having a longitudinal axis A; a partition member 3 housed inside tank 2; and a supporting structure 4 for supporting tank 2 on a floor surface P.

Tank 2 comprises a wall 5 and a hatch 6 defining a chamber 7, in which hatch 6 permits any type of cleaning or maintenance work; an inlet 8 by which both liquid phase L and organic material S are fed into chamber 7; an outlet 9 from which to withdraw organic material S; and an outlet 10 from which to withdraw liquid phase L from chamber 7.

Partition member 3 comprises a plate 11 in the form of a cylindrical sector, the cross section of which is symmetrical with respect to axis A (Figure 2). More specifically, plate 11 is positioned with a peripheral edge 12 hermetically contacting wall 5 and hatch 6, and has a number of holes 13 permitting passage of liquid phase L and preventing passage of organic material S.

That is, plate 11 divides chamber 7 into a first compartment 14 defined by the portion underneath plate 11 and for receiving, in use, both liquid phase L and organic material S; and a second compartment 15 defined by the portion over plate 11 and for receiving, in use, liquid phase L and air.

In actual use, and as shown in Figures 1 and 2, organic material S is fed into compartment 14, and a liquid phase L is fed into chamber 7 up to a level over the highest point of plate 11, so that liquid phase L occupies the whole of first compartment 14 and part of second compartment 15, while organic material S is packed beneath plate 11. Organic material S has a lower specific weight than liquid phase L and so tends to float to the top, but is kept immersed in liquid phase L by plate 11, as shown in Figures 1 and 2.

Organic material S is thus kept fully immersed in liquid phase L to prevent oxidation and aerobic fermentation typical of the known state of the art, but without mechanically stressing organic material S, thus preventing undesired substances from solubilizing in and so impairing the organoleptic characteristics of liquid phase L.

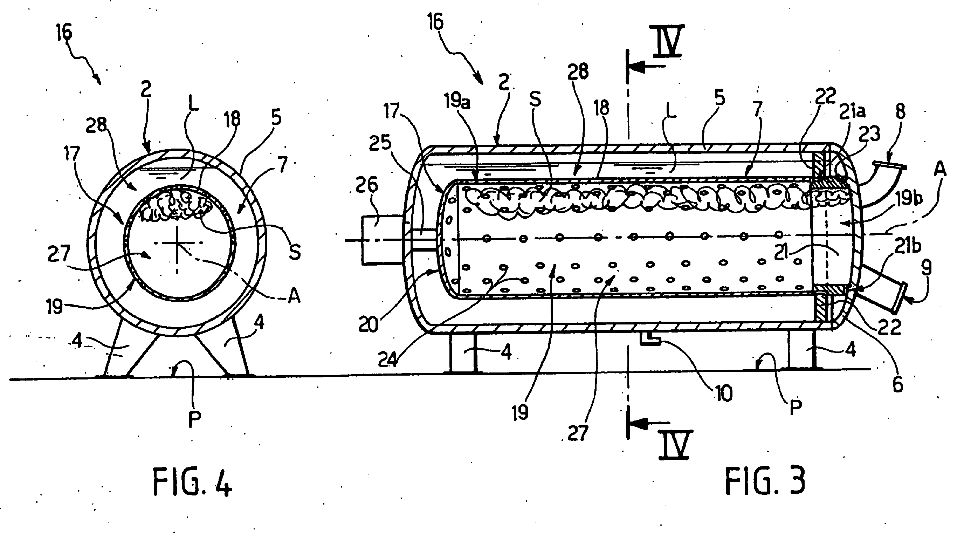

Number 16 in Figures 3 and 4 indicates as a whole a second embodiment of the device according to the present invention, any component parts of which similar to those of device 1 are indicated using the same reference numbers with no further description.

Device 16 comprises a partition member 17, in turn comprising a cylindrical cage 18 housed inside chamber 7 and coaxial with axis A. Cage 18 is defined by a cylindrical lateral wall 19, and a convex circular wall 20 closing one end 19a of cage 18. At one opposite end 19b to end 19a, cage 18 comprises an annular portion 21 having a cylindrical radial face 21a fitted with four pads 22 (only two shown in Figure 3) located between wall 5 and portion 21, and an annular axial face 21b hermetically contacting a respective annular portion 23 of hatch 6.

Pads 22 provide for both supporting and permitting rotation of cage 18 about axis A.

Cage 18 thus has one end closed by convex wall 20, and one end with no wall but closed by hatch 6, so that, in this embodiment, the same member 6 acts as a hatch for both tank 2 and cage 18.

Both cylindrical lateral wall 19 and circular wall 20 have a number of holes 24 permitting passage of liquid phase L and preventing passage of organic material S.

A rotation shaft 25 extends outwards of cage 18 from circular wall 20, and is connected to a known motor reducer 26 (not described in detail) for rotating cage 18 about axis A.

In other words, cage 18 divides chamber 7 into a first compartment 27 inside cage 18 and for receiving, in use, organic material S and liquid phase L; and a second compartment 28 outside cage 18 and for receiving, in use, liquid phase L and air. That is, compartment 28 is located between cage 18 and wall 5 and hatch 6 of tank 2.

In actual use, organic material S is fed into compartment 27, and liquid phase L is fed into chamber 7 to submerge cage 18, so that liquid phase L occupies the whole of first compartment 27 and part of second compartment 28, while organic material S is packed inside compartment 27 in cage 18. As stated previously, organic material S tends to float, but is kept immersed in liquid phase L by cage 18.

As stated previously, organic material S is thus kept fully immersed in liquid phase L, with all the advantages referred to with reference to device 1.

Motor reducer 26 provides for shifting organic material S with respect to liquid phase L. And, since organic material S is fully immersed in liquid phase L, thus preventing any of the drawbacks posed by oxidation of organic material S, cage 18 is rotated extremely slowly to prevent subjecting organic material S to mechanical stress.

To simplify displacement of organic material S inside cage 18, lateral wall 19 may comprise a helical projection facing compartment 27. Such a solution is known in the technical field to which the invention relates, and mainly provides for loading and unloading organic material S easily in and out of cage 18.

## Claims

1. A device (1; 16) for processing fruit (S) in a liquid phase (L); the device (1; 16) comprising a tank (2) having a wall (5) and a hatch (6) defining a chamber (7), a partition member (3; 17), which is housed inside said chamber (7); said partition member (3; 17) divides the chamber (7) into a first compartment (14; 27) housing fruit and a liquid phase (L), and a second compartment (15; 28) only housing the liquid phase (L), and comprises a number of openings (13; 24) permitting passage of the liquid phase (L) between the first (14; 27) and the second (15; 28) compartment; said device being **characterised in that** said partition member (3; 17) is placed horizontally so that said first compartment (14; 27) is located beneath the second compartment (15; 28) and said fruit is totally immersed in said liquid phase (L), and that said first compartment (14; 27) is completely separated from said second compartment (15; 28) by said partition member (3; 17), in the manner that said first compartment (14; 27) communicates with said second compartment (15; 28) only through said openings (13; 24) of said partition member (3; 17); said openings (13; 24) having a size to permit liquid phase only to pass.

2. A device as claimed in Claim 1, **characterized in that** said partition member (3) comprises a plate (11) having a number of holes (13), and an edge (12) contacting said wall (5) and said hatch (6).

3. A device as claimed in Claim 1, **characterized in that** said partition member (17) comprises a cage (18) having a number of holes (24); said first compartment (27) being defined inside said cage (18), and said second compartment (28) being defined between the cage (18) and the wall (5) and hatch (6) of said tank (2).

4. A device as claimed in Claim 3, **characterized in that** said cage (18) rotates about a respective longitudinal axis (A).

5. A device as claimed in Claim 4, **characterized in that** said cage (18) comprises an end portion (21) having a radial face (21a), and an axial face (21b) hermetically contacting a respective annular portion (23) of said hatch (6).

## Patentansprüche

1. Vorrichtung (1, 16) zur Behandlung von Früchten (S) in einer flüssigen Phase (L), wobei die Vorrichtung (1, 16) einen Kessel (2), welcher eine Wandung (5) und eine Klappe (6) aufweist, die eine Kammer (7) bilden, und ein Trennungsglied (3, 17) umfasst, welches die Kammer (7) in ein erstes Abteil (14, 27), das Früchte und eine flüssige Phase (L) aufnimmt, und ein zweites Abteil (15, 28), das nur die flüssige Phase (L) aufnimmt, aufteilt, und welches eine Anzahl von Öffnungen (13, 24) umfasst, die den Durchtritt der flüssigen Phase (L) zwischen dem ersten (14, 27) und dem zweiten (15, 28) Abteil erlauben, aufweist, wobei die Vorrichtung **dadurch gekennzeichnet ist, dass** das Trennungsglied (3, 17) waagerecht angeordnet ist, so dass das erste Abteil (14, 27) unterhalb des zweiten Abteils (15, 28) angeordnet ist und die Früchte vollständig in die flüssige Phase (L) eingetaucht sind, und dass das erste Abteil (14, 27) vollständig vom zweiten Abteil (15, 28) durch das Trennungsglied (3, 17) getrennt ist, derart, dass das erste Abteil (14, 27) mit dem zweiten Abteil (15, 28) nur durch die Öffnungen (13, 24) des Trennungsgliedes (3, 17) in Verbindung steht, wobei die Öffnungen (13, 24) eine Größe aufweisen, um nur der flüssige Phase den Durchtritt zu erlauben.

2. Vorrichtung gemäß Anspruch 1, **dadurch gekennzeichnet, dass** das Trennungsglied (3) eine Platte (11) umfasst, welche eine Anzahl von Löchern (13) aufweist und eine Kante (12), welche die Wandung (5) und die Klappe (6) kontaktiert.

3. Vorrichtung gemäß Anspruch 1, **dadurch gekennzeichnet, dass** das Trennungsglied (17) einen Käfig (18) umfasst, welcher eine Anzahl von Löchern (24) aufweist, wobei das erste Abteil (27) innerhalb des Käfigs (18) definiert wird und das zweite Abteil (28) zwischen dem Käfig (18) und der Wandung (5) und der Klappe (6) von dem Kessel (2) definiert wird.

4. Vorrichtung gemäß Anspruch 3, **dadurch gekennzeichnet, dass** der Käfig (18) um eine zugehörige Längsachse (A) rotiert.

5. Vorrichtung gemäß Anspruch 4, **dadurch gekennzeichnet, dass** der Käfig (18) ein Endstück (21) umfasst, welches eine radiale Fläche (21a) aufweist und eine axiale Fläche (21b), die ein zugehöriges Ringstück (23) der Klappe (6) hermetisch kontaktiert.

## Revendications

1. Dispositif (1 ; 16) pour le traitement de fruits (S) dans une phase liquide (L), le dispositif (1 ; 16) comprenant un réservoir (2) ayant une paroi (5) et une trappe (6) définissant une chambre (7), un élément de séparation (3 ; 17), qui est reçu à l'intérieur de ladite chambre (7) ; ledit élément de séparation (3 ; 17) divise la chambre (7) en un premier compartiment (14 ; 27) recevant les fruits et une phase liquide (L), et un second compartiment (15 ; 28) recevant uniquement la phase liquide (L), et comprend un certain nombre d'ouvertures (13 ; 24) permettant le passage de la phase liquide (L) entre le premier (14 ; 27) et le second (15 ; 28) compartiment ; ledit dispositif étant **caractérisé en ce que** ledit élément de séparation (3 ; 17) est placé horizontalement de sorte que ledit premier compartiment (14 ; 27) est situé sous le second compartiment (15 ; 28) et lesdits fruits sont totalement immergés dans la phase liquide (L) et que ledit premier compartiment (14 ; 27) est totalement séparé dudit second compartiment (15 ; 28) par ledit élément de séparation (3 ; 17), de telle manière que ledit premier compartiment (14 ; 27) communique avec ledit second compartiment (15 ; 28) uniquement à travers lesdites ouvertures (13 ; 24) dudit élément de séparation (3 ; 17) ; lesdites ouvertures (13 ; 24) ayant une taille permettant à la phase liquide seule de passer.

2. Dispositif selon la revendication 1, **caractérisé en ce que** ledit élément de séparation (3) comprend une plaque (11) ayant un certain nombre de trous (13) et un bord (12) en contact avec ladite paroi (5) et ladite trappe (6).

3. Dispositif selon la revendication 1, **caractérisé en ce que** ledit élément de séparation (17) comprend une cage (18) ayant un certain nombre de trous (24) ; ledit premier compartiment (27) étant défini à l'intérieur de ladite cage (18) et ledit second compartiment (28) étant défini entre la cage (18) et la paroi (5) et la trappe (6) dudit réservoir (2).

4. Dispositif selon la revendication 3, **caractérisé en ce que** ladite cage (18) tourne autour d'un axe longitudinal respectif (A).

5. Dispositif selon la revendication 4, **caractérisé en ce que** ladite cage (18) comprend une partie d'extrémité (21) ayant une face radiale (21a) et une face axiale (21b) en contact hermétique avec une partie annulaire respective (23) de ladite trappe (6).
